# EUROPÄISCHE PATENTSCHRIFT

(11) **EP 1 083 838 B1**
(45) Veröffentlichungstag und Bekanntmachung des Hinweises auf die Patenterteilung: **23.04.2003**
(21) Anmeldenummer: 00903424.0
(22) Anmeldetag: 04.02.2000
(51) Int. Cl.: A61B 18/02

(54) **VORRICHTUNG FÜR KRYOCHIRURGISCHE EINGRIFFE, INSBESONDERE FÜR DIE TUMORBEHANDLUNG**
DEVICE FOR CARRYING OUT CRYOSURGICAL INTERVENTIONS, ESPECIALLY FOR TREATING TUMORS
DISPOSITIF POUR INTERVENTIONS CRYOCHIRURGICALES, EN PARTICULIER POUR LE TRAITEMENT DE TUMEURS

(30) Priorität: 12.02.1999 AT 20399; 29.03.1999 AT 57199; 27.04.1999 AT 74199; 28.04.1999 AT 75399; 29.04.1999 AT 76799
(43) Veröffentlichungstag der Anmeldung: 21.03.2001
(73) Patentinhaber: Korpan, Nikolai, 1190 Wien (AT); Zharkov, Jaroslav, Kiew, 252146 (UA)
(72) Erfinder: Korpan, Nikolai, 1190 Wien (AT); Zharkov, Jaroslav, Kiew, 252146 (UA)
(74) Vertreter: Babeluk, Michael, Dipl.-Ing. Mag.
(86) Internationale Anmeldenummer: AT0000025
(87) Internationale Veröffentlichungsnummer: WO00047121

(56) Entgegenhaltungen:
- WO-A-97/14005
- FR-A- 2 429 988
- FR-A- 2 727 618
- US-A- 5 324 286

## Beschreibung

Die Erfindung betrifft eine kryogene Vorrichtung gemäß dem Oberbegriff des Anspruches 1.

Kryochirurgische Instrumente finden überwiegend in der Krebsbehandlung Anwendung, wobei die Kryoinstrumente an der Oberfläche bzw. im Inneren des tumorbefallenen Gewebes appliziert werden, so dass infolge gezielter extremer Kälteeinwirkung das pathologische Gewebe weitgehend destruiert bzw. avitalisiert wird und sowohl die biologisch-chemischen als auch biologisch-physikalischen Prozesse lokal irreversibel werden. Dadurch werden kleinere Tumormassen zerstört und die Krebszellen können nicht mehr weiter wachsen. Mit dieser Maßnahme werden sowohl das Lokalrezidiv als auch die Metastasierung von Krebszellen bei kleinen Tumoren verhindert. Als weitere Anwendungsgebiete bieten sich beispielsweise Allgemeinchirurgie, Urologie, Gynäkologie, HNO- und Augenkrankheiten, Plastische Chirurgie, Kieferchirurgie, Orthopädie, Veterinärmedizin aber auch Phytopathologie etc. an.

Nachteilig bei diesen bekannten Kryoinstrumenten ist jedoch, dass die großen Tumorgewebe nicht zur Gänze zerstört werden.

Ebenso ist nachteilig bei den bekannten Vorrichtungen, dass die Strömungswege für das flüssige kryogene Medium und das gasförmige kryogene Medium nicht voneinander getrennt sind, dadurch werden sich die Strömungen der einzelnen Phasen beeinflussen. Es ist damit nicht möglich, die Konstanthaltung der Minimaltemperatur an der Außenseite der Arbeitsfläche der Vorrichtungen zu erreichen und den Verlust des kryogenen Mediums gering zu halten.

Die FR 2 727 618 A beschreibt eine kryogene Vorrichtung gemäß Oberbegriff des Auspruchs 1, bei der das Endstück eine mit Rippen versehene Fläche aufweist, die mit einer porigen Struktur verbunden ist. Der Wirkungsgrad einer solchen Vorrichtung ist zwar gut, jedoch für bestimmte Anwendungen nicht ausreichend, so dass beispielsweise große pathologische Tumorgewebe nicht vollständig destruiert werden können.

Daher ist es Aufgabe der Erfindung, die bekannten Instrumente dahingehend zu verbessern, dass auch große pathologische Tumorgewebe zur Gänze destruiert bzw. avitalisiert werden und sowohl die biologisch-chemischen als auch biologisch-physikalischen Prozesse irreversibel werden. Dadurch wird die gesamte Tumormasse zerstört und die Krebszellen können nicht mehr weiter wachsen, so dass ein Lokalrezidiv (Nachwachstum) des Tumors vermieden, als auch die Metastasierung von Krebszellen sowohl bei kleinen als auch bei großen Tumoren verhindert werden.

Außerdem wird zur Konstanthaltung der Minimaltemperatur an der Außenseite des Endstückes der Instrumente für die kryogene Wirkung der Verlust des kryogenen Mediums äußerst gering gehalten.

Dies wird nur ermöglicht durch das Erreichen und Konstanthalten der Minimaltemperatur an der Arbeitsaußenseite des Kryoinstrumentes im Kontakt mit biologischem Gewebe gemäß den Merkmalen des kennzeichnenden Teiles des Anspruches 1.

Die Unteransprüche zeigen kryogene Instrumente, die an einer Verbindungsanordnung eines kryogenen Gerätes bzw. Systems angeschlossen und für unterschiedliche Anwendungsgebiete eingesetzt werden.

Der Anspruch 2 zeigt ein als Kryoapplikator ausgebildetes Kryoinstrument, welches sich für die Behandlung des an der Oberfläche bzw. im Inneren großen tumorbefallenen Gewebes infolge der gleichmäßigen Arbeitsfläche, vorzugsweise in Größen zwischen 3 mm und 55 mm, eignet und gezielt appliziert werden kann.

Die Erfindung gemäß Ansprüche 3 bis 6 stellt ein als Kryonadel ausgebildetes Kryoinstrument dar, welches vorzugsweise Anwendung für die Behandlung tiefliegender pathologischer Gewebe, z.B. Leber- oder Prostatatumor, findet.

Anspruch 7 zeigt ein als Kryosonde ausgebildetes Kryoinstrument für die Behandlung von Tumoren, die mit unterschiedlicher Infiltration in das gesunde Gewebe einwachsen, z.B. Weichteilsarkom.

Der Kryoinstrument gemäß Auspruch 8 als Kryomammotom entwickelt, wird bei der Behandlung worzugsweise von Brust-und Prostatakrebs eingesetzt.

Gestielte Tumore, beispielsweise Polypen, werden mit der Kryoklemme (Anspruch 9) gezielt behandelt.

Die Erfindung wird anhand einer schematischen Darstellung der Vorrichtung näher erläutert und in der Zeichnung zeigen
**Fig. 1** ein als Kryoapplikator ausgebildetes Kryoinstrument im Längsschnitt;
**Fig. 2** eine Kryonadel im Längsschnitt;
**Fig. 3** eine Kryonadel im Querschnitt;
**Fig. 4** eine Kryosonde im Querschnitt;
**Fig. 5** ein Kryomammotom im Längsschnitt;
**Fig. 6** ein Kryomammotom gemäß Schnitt A-A;
**Fig. 7** eine Kryoklemme im Längsschnitt;
**Fig. 8** einen Ausschnitt der Fig. 7 - die Spitze der Kryoklemme;
**Fig. 9** ein Detail von Fig. 8 im Querschnitt (nach Pfeil A);
**Fig. 10** ein Detail von Fig. 8 im Längsschnitt (nach Pfeil B).

Die in der Erfindung beanspruchten Kryoinstrumente werden an eine in der Figuren nicht dargestellten Verbindungsanordnung eines kryogenen Gerätes bzw. Systemes angeschlossen.

Die Vorrichtung für kryochirurgische Eingriffe besteht aus einem Gehäuse (1), das aus rostfreiem Stahl besteht und unterschiedliche Querschnittsformen, beispielsweise rund, aufweisen kann, in dem sich je eine Leitung für den Direkt- (2) und Rückstrom (3) eines kryogenen Mediums, z.B. flüssiger Stickstoff, befindet, mit mindestens einem geschlossenen Endstück (4), welches eine Arbeitsfläche (5) mit einer Außenseite (6) für die Abkühlung eines Gewebes bildet und aus einem stark wärmeleitenden sauerstofffreien Kupfer besteht, sowie einem Temperatursensor (7).

Zum Erreichen und Konstanthalten der Minimaltemperatur, insbesondere zwischen -40 °C und -196 °C, mit geringem Verlust des kryogenen Mediums an der Arbeitsaußenseite (6) des Endstückes (4) des Kryoinstrumentes im Kontakt mit biologischem Gewebe, insbesondere Krebsgewebe, ist im Gehäuse (1) ein Element mit einer porigen Struktur (8), beispielsweise aus Kuprum, angeordnet.

Der Grad der Porösität und die Porengröße, in der Dimension von etwa 10 µm bis 15 µm, nehmen von innen nach außen zu; dadurch werden die Kapillarkräfte der porigen Struktur (8) größer als der Dampfdruck des gegebenenfalls an Rippen (9) verdampften kryogenen Mediums. Es wird ausschließlich das verdampfte kryogene Medium, gegebenenfalls durch Kanäle (10) zwischen den Rippen (9), in die kryogene Leitung (3) abgeführt, sodass die flüssige Phase in der porigen Struktur (8) nicht beeinflußt wird.

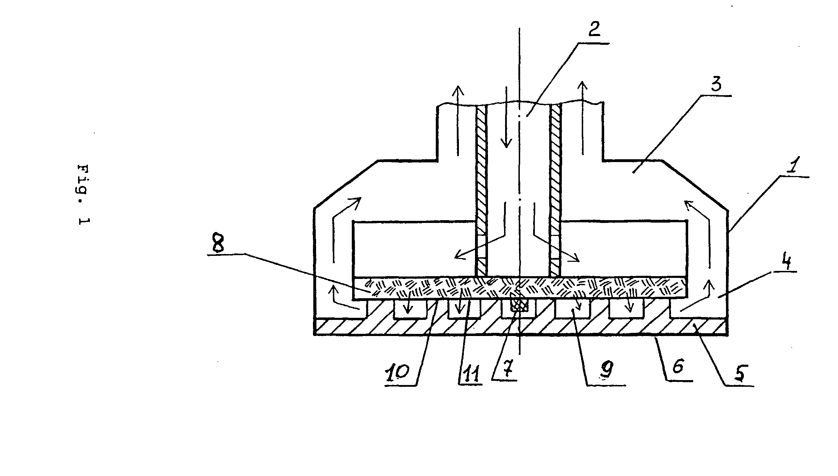

Der Kryoapplikator (**Fig. 1**) besteht aus einem Endstück (4), das gleichmäßig mit senkrecht an der Innenseite angeordneten Rippen (9) ausgestattet ist. Zwischen den Rippen (9) bilden sich Kanäle (10), durch die das verdampfte kryogene Medium in die kryogene Leitung (3) rückgeführt wird.

An der Innenseite der mit Rippen (9) versehenen Fläche ist ein Element mit einer porigen Struktur (8) nach dem Diffusionsschweißverfahren angeschweißt. Die Struktur (8) wird aus fehlorientierten Kupferdrahtabschnitten, die ebenso nach dem Diffusionsschweißverfahren zusammengeschweißt sind, gebildet. Das Porösitätsverhältnis und die Porengrößen sind variabel und etwa 10 µm bis 15µ m groß; sie werden größer, wenn sie sich der Rippenkante (11) nähern. Das flüssige kryogene Medium wird der porigen Struktur (8) zugeführt, eingezogen und infolge der kapillaren Kräfte festgehalten. Das Sieden des flüssigen kryogenen Mediums und die Wärmeabführung vom biologischen Gewebe erfolgen an der Grenze zwischen der porigen Struktur (8) und den Kanten der Rippen (9). Durch das oben angeführte Porositätsverhältnis und die Porengrößen werden die kapillaren Kräfte stärker als der Dampfdruck, sodass ausschließlich das verdampfte kryogene Medium durch die Kanäle (10) zwischen den Rippen (9) in die kryogene Leitung (3) abgeführt wird. Dadurch wird die flüssige Phase in der porigen Schicht (8) nicht beeinflußt.

Das Gehäuse (1) besitzt vorzugsweise einen runden Querschnitt und das Endstück (4) ist als eine mit an der Innenseite angeordneten Rippen (9) versehenen Fläche ausgebildet, sodass zwischen den Rippen (9) Kanäle (10) zum Durchtritt des verdampften kryogenen Mediums gebildet werden. An die Fläche ist eine Schicht mit der porigen Struktur (8) angeschweißt.

Die porige Struktur (8) ist aus fehlorientierten Kupferdrahtabschnitten, die nach dem Diffusionsschweißverfahren zusammengeschweißt sind, gebildet. Das Porösitätsverhältnis und die Größe der Poren werden größer, wenn sie sich der Rippenkante (11) nähern.

Die durch die Erfindung realisierte vollkommene Trennung der Strömungswege des flüssigen kryogenen Mediums und des gasförmigen kryogenen Mediums, wie in **Figur 1** ersichtlich, und durch die relativ geringe Porengröße von etwa 10 µm bis 15 µm wird eine hervorragende Wärmeübertragung gewährleistet, die es ermöglicht, dass die Temperatur an der Außenseite des Kryoinstrumentes (Kryoapplikators) nur geringfügig oberhalb der Siedetemperatur des kryogenen Mediums liegt. Aufgrund der Anordnung der porigen Struktur (8) mit Porengrößen von etwa 10 µm bis 15 µm und weniger werden die kapillaren Kräfte stärker als der Dampfdruck des kryogenen Mediums, sodass ausschließlich das verdampfte kryogene Medium durch die Kanäle (10) zwischen den Rippen (9) in die kryogene Leitung (3) abgeführt und das flüssige kryogene Medium in der unteren Schicht der porigen Struktur (8) gehalten wird. Dadurch wird die flüssige Phase in der porigen Struktur (8) nicht beeinflußt.

Mit den Maßnahmen der Merkmale des Anspruchs 3 wird eine gerichtete und eindeutige Strömung des kryogenen Mediums auch innerhalb des porösen Materials (8) erreicht.

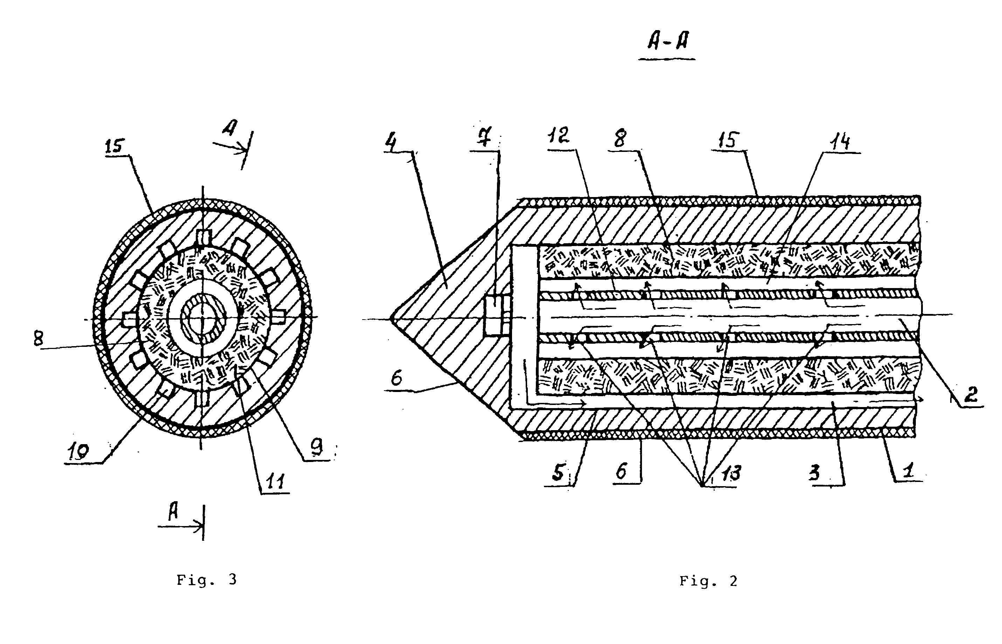

Gemäß **Fig. 2** und **3** ist das Kryoinstrument als hohle Kryonadel ausgebildet. Die Innenseite (12) des im wesentlichen zylindrischen Gehäuses (1) ist mit Rippen (9) versehen. Die Kanäle (10) werden zwischen den Rippen (9) zum Austreten des verdampften kryogenen Mediums somit gebildet, und an den Rippen (9) ist eine runde Schicht mit poriger Struktur (8) angeschweißt. Die Wände der Leitung (2) für den Direktstrom sind in der Länge, die der Gefrierungslänge der Kryonadel entspricht, mit einer Vielzahl von radialen, gegenüberliegenden Ausströmöffnungen (13) versehen, durch die das kryogene Medium über einen Zwischenraum (14), der zwischen der Wand der Leitung (2) für den Direktstrom und der porigen Struktur (8) angeordnet ist, in die porige Struktur (8) einfließt, wodurch eine Durchströmung der porigen Struktur (8) im wesentlichen in Radialrichtung bewirkt wird, sodass die Kapillarkräfte der porigen Struktur (8) stärker als der Dampfdruck des an den Rippen (9) verdampften kryogenen Mediums sind. Die flüssige Phase in der porigen Struktur (8) wird nicht beeinflußt und eine gerichtete eindeutige Strömung des kryogenen Mediums wird auch innerhalb des porösen Materials erreicht.

Die Leitung (2) im Inneren der porigen Struktur (8) ist mit Spiel angeordnet.

Das Gehäuse (1) weist auf seiner Innenseite Rippen (9) auf, die sich im wesentlichen in Axialrichtung erstrecken, und zwischen denen Kanäle (10) zur Rückführung des verdampften kryogenen Mediums ausgebildet sind.

Zwecks Vermeiden des Dampfhäubchens an den Rippen (9) bei der Bildung des Überganges vom flussigen in den dampfförmigen Zustand des kryogenen Mediums, um damit auch eine Minimaltemperatur an der Arbeitsfläche der im wesentlichen zylindrischen Kryonadel zu ermöglichen, wird die Arbeitsfläche des im wesentlichen zylindrischen Gehäuses (1) der Kryonadel von außen mittels einer Wärmeisolation (15) durch eine dünne Schicht, z.B. 0,2-0,7 mm im Kryoinstrument mit Durchmesser vom 4 mm aus einem Wärmeisolierungsmaterial mit einer niedrigen Wärmeleitung, z.B. rostfreier Stahl, angeordnet. Die Schichtdicke ist vom Durchmesser des Kryoinstrumentes abhängig, d.h. je größer der Durchmesser der Kryonadel ist, desto geringer wird die Stärke der Schicht gehalten. Es wird damit die Bedingung für den Übergangszustand des kryogenen Mediums zusätzlich erzielt, wodurch der Wärmestrom zum in der porigen Struktur verdampften kryogenen Medium vermindert und damit die Minimaltemperatur ergänzend erreicht wird.

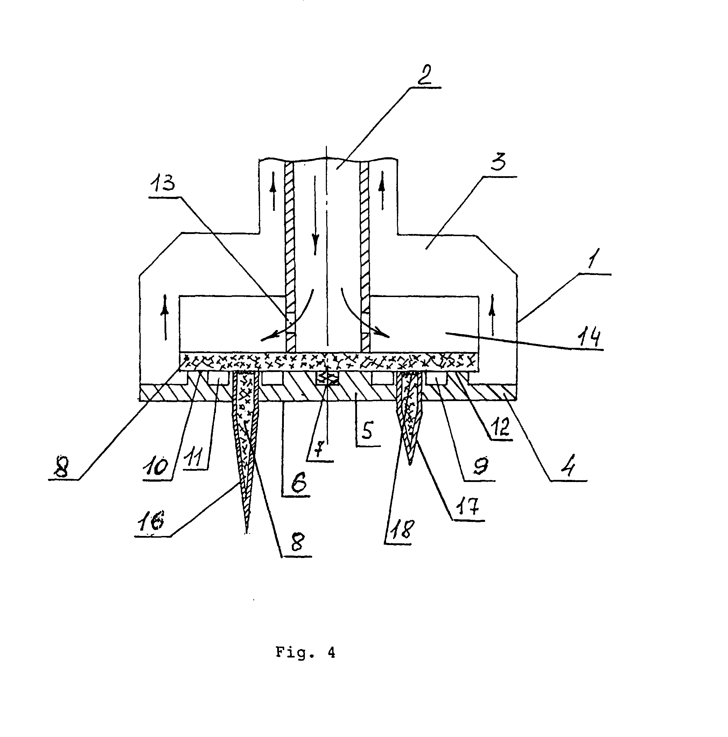

Das kryogene Instrument **(Fig. 4)** ist als Kryosonde ausgebildet, welche Elemente (8) mit der porigen Struktur besitzt. Die Wände der Leitung (2) in der Nähe des Endstückes (4) sind mit gegenüberliegenden Durchtrittsöffnungen (13) versehen, durch die das kryogene Medium in einen Zwischenraum (14) einfließt. An der Innenseite des Endstückes (4) sind Zacken (16) in konischer Form von verschiedener Länge, z.B. von 1 bis 30 cm und unterschiedlicher Anzahl, z.B. von 1 bis 15, angeordnet und innen mit poriger Struktur (8) und außen mit einer undurchlässigen Wand (17) versehen, wobei die Zacken (16) mit Schraubengewinde (18) abnehmbar befestigt sind, zwecks Erreichen und Konstanthalten der Minimaltemperatur im Kontakt nicht nur mit oberflächlich liegendem, sondern auch mit tief liegendem pathologischen Gewebe, womit die Kryodestruktion der ganzen Tumormasse gesichert ist.

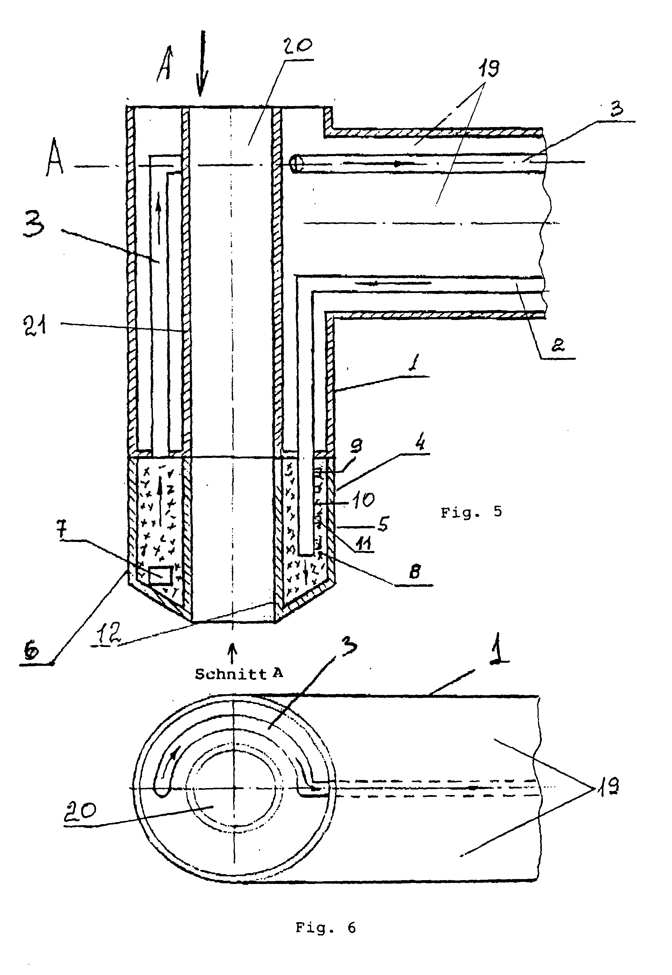

Das kryogene Instrument **(Fig. 5 und 6)** ist als Kryomammotom mit dem Endstück (4) in Form einer Kryospitze ausgebildet, das für Biopsie und Aspiration des pathologischen Gewebes, insbesondere Brusttumorgewebes, im Gehäuse (1) mit einer Vakuumisolation (19), einem wärmeisolierten Kanal (20), vorzugsweise im Durchmesser 6 mm bis 7 mm, mit einer dünnen Wand (21), vorzugsweise von 0.2 mm bis 0.3 mm, aus rostfreiem Stahl, ausgestattet ist. Das Gehäuse (1) ist unter einem Winkel, vorzugsweise von 15° bis 160°, hergestellt. Die Leitung (2) für den Direktstrom des kryogenen Mediums führt in das Element mit poriger Struktur (8) hinein. Die Innenseite (12) der Kryospitze (4) gefriert einen Rest des pathologischen Gewebes, welches sowohl im Kanal (20) als auch im Organ, z.B. in der Brust, nach der Aspiration übrig bleibt. Die Leitung (3) für den Rückstrom des kryogenen Mediums ist mit dem Element mit poriger Struktur (8) nicht verbunden und umgibt den Kanal (20) halbkreisförmig. Der Kanal (20) ist für verschiedene Funktionen, insbesondere Biopsie und Aspiration des pathologischen Gewebes, stets frei gehalten.

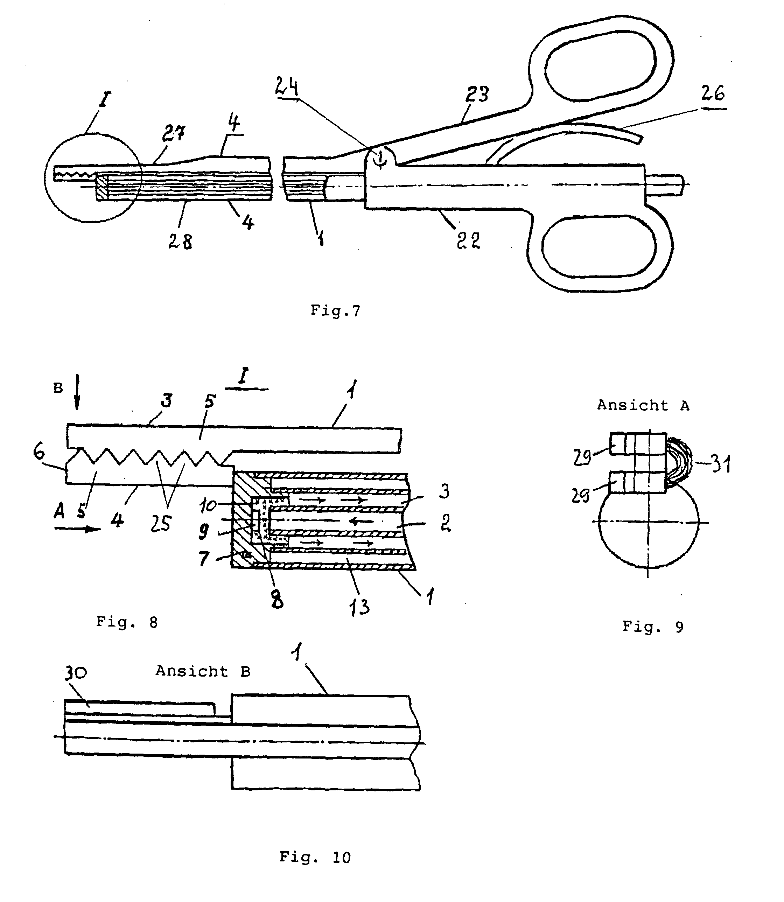

Gemäß **Fig. 7 bis 10** ist das Kryoinstrument als Kryoklemme mit einem Gehäuse (1) ausgebildet. Das Gehäuse (1) ist mit einer unbeweglichen und beweglichen Branche (22, 23), die durch ein Gelenk (24) miteinander verbunden sind, deren Arbeitsflächen (5) mit der Außenseite (6) mit Zacken (25) ausgestattet sind, angeordnet, wobei das Gehäuse (1) aus rostfreiem Stahl mit niedriger Wärmeleitung, und die Branche (22, 23) aus Cuprum mit hoher Wärmeleitung hergestellt werden. Die Kryoklemme besteht ferner aus einem Feder-Stopper (26) für die bewegliche Branche (23) sowie einem Maul mit einem beweglichen und unbeweglichen Teil (27, 28). An der Innenseite des beweglichen (27) und unbeweglichen (28) Maulteiles befindet sich ein wärmeisolierter Erhitzer (29). Ein Wärmeaustauscher (30) ist mit dem unbeweglichen Maulteil (28), der nicht mit Zacken (25) versehen ist, verbunden. Eine Brücke (31) aus einem elastischen Kälteleiter, der aus einem Draht, vorzugsweise im Durchmesser 4 bis 8 mm, welcher aus mehreren Fädern, vorzugsweise im Durchmesser 20 bis 60 µm, aus Cuprum ausgebildet ist, ist zwischen dem Wärmeaustauscher (30) und dem beweglichen Maulteil (28) angeordnet, zwecks Wärmeableitung vom beweglichen Maulteil (27) in den Wärmeaustauscher (30). Die Leitung (2) für den Direktstrom des kryogenen Mediums und die Leitung (3) für den Rückstrom des kryogenen Mediums sind im unbeweglichen Maulteil (28) angeordnet, zwecks der präzisen und gesicherten Kryodestruktion des gestielten pathologischen Gewebes, insbesondere Krebsgewebe, in einem anatomischen Bereich, z.B. im Rektum, sodass ein Rezidiv des Tumors vermieden wird und nur die Erwärmung der gesunden benachbarten Strukturen erfolgt, womit das gesunde Gewebe geschont wird.

## Patentansprüche

1. Kryoinstrument, als Kryoapplikator ausgebildet, für kryochirurgische Eingriffe im human- und veterinärmedizinischen Bereich, insbesondere in der Tumorbehandlung, sowie in der Phytopathologie zum Anschluss an einer Verbindungsanordnung eines kryogenen Gerätes bzw. Systems, bestehend aus einem Gehäuse (1), welches vorzugsweise einen runden Querschnitt besitzt, in dem sich je eine Leitung für den Direkt- (2) und Rückstrom (3) eines kryogenen Mediums, z.B. flüssiger Stickstoff, befindet, mit mindestens einem geschlossenen Endstück (4), welches eine Arbeitsfläche (5) mit einer Außenseite (6) für die Abkühlung eines Gewebes bildet, sowie einem Temperatursensor (7), wobei das Endstück (4) als eine mit an der Innenseite angeordneten Rippen (9) versehene Fläche, an die eine Schicht mit einer porigen Struktur (8) angeschweißt ist, ausgebildet ist, so dass zwischen den Rippen (9) Kanäle (10) zum Durchtritt des werdampften kryogenen Mediums gebildet werden, **dadurch gekennzeichnet, dass** zum Erreichen und Konstanthalten der Minimaltemperatur, insbesondere -40°C und -196°C, mit geringem Verlust des kryogenen Mediums an der Arbeitsaußenseite des Kryoiristrumenstes im Kontakt mit biologischem Gewebe, insbesondere Krebsgewebe, im Gehäuse (1) das Element mit einer porigen Struktur (8), beispielsweise aus Cuprum, deren Grad der Porosität und Porengröße in der Dimension von etwa 10 µm bis 15 µm, von innen nach außen, das heißt von der stromaufwärtigen Seite zur stromabwärtigen Seite, zunimmt, angeordnet ist, so dass die Kapillarkräfte der porigen Struktur (8) größer als der Dampfdruck des gegebenenfalls an Rippen (9) verdampften kryogenen Mediums sind, und ausschließlich das verdampfte kryogene Medium, durch die Kanäle (10) zwischen den Rippen (9), in die kryogene Leitung für den Rückstrom (3) abgeführt wird, so dass die flüssige Phase in der porigen Schicht (8) nicht beeinflusst wird.

2. Kryoinstrument nach Anspruch 1, **dadurch gekennzeichnet, dass** die Struktur (8) aus fehlorientierten Kupferdrahtabschnitten, die nach dem Diffusionsschweißverfahren zusammengeschweißt sind, gebildet ist.

3. Kryoinstrument nach Anspruch 1, **dadurch gekennzeichnet, dass** es als hohle Kryonadel, vorzugsweise mit einer Länge bis 150 mm und einer Breite von 3 bis 15 mm, ausgebildet ist, wobei die Innenseite (12) des im Wesentlichen zylindrischen Gehäuses (1) mit Rippen (9) versehen ist, sodass die Kanäle (10) zwischen den Rippen (9) zum Austreten des verdampften kryogenen Mediums gebildet werden, und an den Rippen (9) einen runde Schicht mit poriger Struktur (8) angeschweißt ist, und die Wände der Leitung (2) für den Direktstrom in der Länge, die der Gefrierungslänge der Kryonadel entspricht, mit einer Vielzahl von radialen gegenüberliegenden Ausströmungsöffnungen (13) versehen sind, durch die das kryogene Medium über einen Zwischenraum (14), der zwischen der Wand und der Leitung (2) für den Direktstrom und der porigen Struktur (8) zugeordnet ist, in die porige Struktur (8) einfließt.

4. Kryoinstrument nach Anspruch 3, **dadurch gekennzeichnet, dass** die Leitung (2) im inneren der porigen Struktur (8) mit Spiel angeordnet ist.

5. Kryoinstrument nach Anspruch 3 oder 4, **dadurch gekennzeichnet, dass** das Gehäuse (1) auf seiner Innenseite (12) Rippen (9) aufweist, die sich im Wesentlichen in Axialrichtung erstrecken, zwischen denen Kanäle (10) zur Rückführung des verdampften kryogenen Mediums ausgebildet sind.

6. Kryoinstrument nach einem der Ansprüche 3 bis 5, **dadurch gekennzeichnet, dass** zwecks Vermeiden des Dampfhäubchens an den Rippen (9) bei der Bildung des Überganges vom flüssigen in den dampfförmigen Zustand des kryogenen Mediums, um damit auch eine Minimaltemperatur an der Arbeitsfläche der im Wesentlichen zylindrischen Kryonadel zu ermöglichen, die Arbeitsfläche des im Wesentlichen zylindrischen Gehäuses (1) der Kryonadel von außen mittels einer Wärmeisolation (15) durch eine dünne Schicht, z.B. 0,2-0,7 mm im Kryoinstrument mit Durchmesser von 4 mm, versehen ist, wobei bei zunehmendem bzw. abnehmendem Durchmesser der Kryonadel die Stärke der Schicht geringer bzw. größer gehalten ist, und ferner aus einem Wärmeisolierungsmaterial mit einer niedrigen Wärmeleitung, z.B. rostfreiem Stahl, damit die Bedingung für den Übergangszustand des kryogenen Mediums zusätzlich erzielt wird, wodurch der Wärmestrom zum in der porigen Struktur (8) verdampften kryogenen Medium vermindert und damit die Minimaltemperatur ergänzend erreicht wird.

7. Kryoinstrument nach Anspruch 1, **dadurch gekennzeichnet, dass** es als Kryosonde ausgebildet ist und die Elemente (8) eine porige Struktur besitzen, wobei die Wände der Leitung (2) in der Nähe des Erdstückes (4) mit gegenüberliegenden Durchtrittsöffnungen (13) versehen sind, durch die das kryogene Medium in einen Zwischenraum (14) einfließt, das an der Innenseite (12) des Endstückes (4) Zacken (16) in konischer Form von verschiedener Länge, z. B. von 1 bis 30 cm und unterschiedlicher Anzahl, z.B. von 1 bis 15, angeordnet und innen mit poriger Struktur (8) und außen mit einer undurchlässigen Wand (17) versehen, und die Zacken (16) mit Schraubgewinde (18) abnehmbar befestigt sind.

8. Kryoinstrument nach Anspruch 1, **dadurch gekennzeichnet, dass** es als Kryomammotom mit dem Endstück (4) in Form einer Kryospritze ausgebildet ist, das für Biopsie und Aspiration des pathologischen Gewebes, insbesondere Brusttumorgewebes, im Gehäuse (1) mit einer Vakuumisolation (19), einem wärmeisolierten Kanal (20), vorzugsweise im Durchmesser 6 mm bis 7 mm, mit einer dünnen Wand (21), vorzugsweise von 0.2 mm bis 0.3 mm, aus rostfreiem Stahl, ausgestattet ist, ferner das Gehäuse (1) unter einem Winkel, vorzugsweise von 15° bis 160°, hergestellt ist, und die Leitung (2) für den Direktstrom des kryogenen Mediums in das Element mit poriger Struktur (8) hineinführt, sodass die Innenseite (12) der Kryospitze (4) einen Rest des pathologischen Gewebes, welches sowohl im Kanal (20) als auch im Organ, z.B. in der Brust, nach der Aspiration übrig bleibt, gefriert, dass die Leitung (3) für den Rückstrom des kryogenen Mediums mit dem Element mit poriger Struktur (8) nicht verbunden ist und den Kanal (20) halbkreisförmig umgibt, sodass der Kanal (20) für verschiedene Funktionen, insbesondere Biopsie und Aspiration des pathologischen Gewebes, stets frei gehalten ist.

9. Kryoinstrument nach Anspruch 1, **dadurch gekennzeichnet, dass** es als Kryoklemme mit einem Gehäuse (1), welches mit einer unbeweglichen und beweglichen Branche (22, 23), die durch ein Gelenk (24) miteinender verbünden sind, deren Arbeitsflächen (5) mit der Außenseite (6) mit Zacken (25) ausgestattet sind, ausgebildet sind, sowie mit einem Maul mit einem beweglichen und unbeweglichen Teil (27, 28) wobei die Leitung (2) für den Direktstrom des kryogenen Mediums und die Leitung (3) für den Rückstrom des kryogenen Mediums im unbeweglichen Maulteil (28) angeordnet sind, dass sich an der Innenseite des beweglichen (27) und des unbeweglichen (28) Maulteils ein wärmeisolierter Erhitzer (29) befindet, und dass der unbewegliche Maulteil (28), der nicht mit Zacken (25) versehen ist, mit einem Wärmeaustauscher (30) verbunden ist, sodass eine Brücke (31) aus einem elastischen Kälteleiter, der aus einem Draht, vorzugsweise im Durchmesser 4 bis 8 mm, welcher aus mehren Federn, vorzugsweise im Durchmesser 20 bis 60 um, aus Cuprum ausgebildet ist, und zwischen dem Wärmetauscher (30) und beweglichen Maulteils (27), angeordnet ist.

## Claims

1. A cryoinstrument, configured as a cryoapplicator for carrying out cryosurgical interventions in the area of human and veterinary medicine, especially for treating tumours, and in the area of phytopathology for connection to a connecting device of a cryogenic appliance or system, comprising a housing (1) the cross section of which is preferably round and in which there is located one line for the direct flow (2) and one line for the return flow (3) of a cryogenic medium, e.g., liquid nitrogen, with at least one closed end piece (4) which forms a working surface (5) with an exterior side (6) for cooling a tissue, and with a temperature sensor (7), the end piece (4) being configured as a surface which is fitted with ribs (9) arranged on the inner side thereof and onto which a layer with a porous structure (8) is welded, so that channels (10) for the passage of the evaporated cryogenic medium are forming between the ribs (9), **characterized in that**, in order to reach the minimum temperature constant and keep it constant, in particular between -40°C and -196°C, with a low loss of the cryogenic medium on the working exterior side of the cryoinstrument which is in contact with the biological tissue, especially cancer tissue, the element with a porous structure (8), made of cuprum for example, and whose degree of porosity and pore size, in the dimension of approximately 10 µm to 15 µm, increases from the interior in a direction toward the exterior, meaning from the upstream side toward the downstream side, is arranged in the housing (1) so that the capillary forces of the porous structure (8) are greater than the vapour pressure of the cryogenic medium possibly evaporated on ribs (9) and that the evaporated cryogenic medium alone is evacuated through the channels (10) between the ribs (9), into the cryogenic line for the return flow (3), so that the liquid phase in the porous layer (8) is not affected.

2. The cryoinstrument according to claim 1, **characterized in that** the structure (8) is made of misoriented copper wire sections which are welded together according to the diffusion welding process.

3. The cryoinstrument according to claim 1, **characterized in that** it is designed as a hollow cryoneedle, having a preferred length of up to 150 mm and a width of 3 to 15 mm, the inner side (12) of the substantially cylindrical housing (1) being provided with ribs (9) so that the channels (10) are formed between the ribs (9) and serve for the exit of the evaporated cryogenic medium and a round layer with a porous structure (8) is welded on the ribs (9) and the walls of the line (2) for the direct flow are provided, in a length mating the freezing length of the cryoneedle, with a plurality of radial opposing exit openings (13) through which the cryogenic medium flows into the porous structure (8) after having passed a space (14) affected to the porous structure (8) and arranged between the wall and the line (2) for the direct flow.

4. The cryoinstrument according to claim 3, **characterized in that** the line (2) within the porous structure (8) is arranged with play.

5. The cryoinstrument according to claim 3 or 4, **characterized in that** the housing (1) is provided on its inner side (12) with ribs (9) substantially extending in axial direction, channels (10) being designed therein between for returning the evaporated cryogenic medium.

6. The cryoinstrument according to one of the claims 3 to 5, **characterized in that,** in order to avoid the steam dome on the ribs (9) as transition from the liquid to the vapour state of the cryogenic medium takes place and to thus also permit a minimum temperature at the working surface of the substantially cylindrical cryoneedle, the working surface of the substantially cylindrical housing (1) of the cryoneedle is provided from the exterior by means of a heat insulation (15) with a thin layer e.g., 0,2 - 0,7 mm in the cryoinstrument with a diameter of 4 mm, wherein the thickness of the layer is smaller or greater as the diameter of the cryoneedle increases or decreases, and furthermore is made of a thermally insulating material with a low thermal conductivity, e.g., stainless steel, in order to additionally achieve the condition for the transition state of the cryogenic medium, the heat flow toward the cryogenic medium evaporated in the porous structure (8) being reduced as a result, which contributes to reach the minimum temperature.

7. The cryoinstrument according to claim 1, **characterized in that** it is designed as a cryoprobe and that the elements (8) have a porous structure, wherein the walls of the line (2) in the vicinity of the end piece (4) are provided with opposite passage holes (13) through which the cryogenic medium flows into a space (14), that notches (16) of a conical shape and of various length, ranging from 1 to 30 cm for example, and number, e.g., from 1 to 15, are arranged on the interior side (12) of the end piece (4) and are provided within with a porous structure (8) and on the outside with an impermeable wall (17), the notches (16) with screw thread (18) being removably fastened.

8. The cryoinstrument according to claim 1, **characterized in that** it is designed as a cryomammotome with the end piece (4) configured to form a cryotip, which is fitted in the housing (1) with a vacuum insulation (19), a heat-insulated channel (20) of a diameter of preferably 6 mm to 7 mm, with a thin wall (21) of preferably 0.2 mm to 0.3mm made of stainless steel for biopsy and aspiration of the diseased tissue, more specifically of tissue from breast tumour, that moreover the housing (1) is manufactured under an angle of preferably 15° to 160° and the line (2) for the direct flow of the cryogenic medium leads into the element with porous structure (8) so that the inner side (12) of the cryotip (4) freezes a remnant of the diseased tissue which remains in the channel (20) as well as in the organ, e.g., in the breast, after aspiration, that the line (3) for the return flow of the cryogenic medium is not connected with the element with porous structure (8) and surrounds the channel (20) describing a semicircle so that the channel (20) is constantly kept free for various functions, more specifically for biopsy and aspiration of the diseased tissue.

9. The cryoinstrument according to claim 1, **characterized in that** it is designed as a cryoclamp with a housing (1) which is provided with an immovable and a movable branch (22, 23) which are connected to one another by a joint (24) and whose working surfaces (5) are fitted on their exterior side (6) with notches (25), as well as with a mouth with a movable and an immovable part (27, 28), the line (2) for the direct flow of the cryogenic medium and the line (3) for the return flow of the cryogenic medium being arranged in the immovable part of the mouth, that a heat-insulated heater (29) is located on the inner side of the movable (27) and immovable (28) part of the mouth and that the immovable part of the mouth (28), which is not provided with notches (25), is connected to a heat exchanger (30) so that a bridge (31), made of an elastic conductor of cold, configured as a wire having a diameter of preferably 4 to 8 mm and consisting of several strands of a diameter of preferably 20 to 60 µm of cuprum, is arranged between the heat exchanger (30) and the movable part of the mouth (27).

## Revendications

1. Instrument cryogénique, réalisé sous la forme d'un applicateur cryogénique pour interventions cryochirurgicales dans le domaine de la médecine humaine et vétérinaire, en particulier pour le traitement des tumeurs, ainsi qu'en phytopathologie, destiné à être relié à un dispositif de raccordement d'un appareil ou système cryogénique, composé d'un corps (1) qui possède de préférence une section ronde, dans lequel se trouve une conduite pour chacun des courants d'aller (2) et de retour (3) d'un milieu cryogénique, par exemple d'azote liquide, et comprenant au moins un embout fermé (4) qui forme une surface active (5) possédant un côté extérieur (6) prévu pour le refroidissement d'un tissu, ainsi qu'un capteur de température (7), l'embout (4) étant constitué par une surface munie de nervures (9) disposées sur le côté intérieur et à laquelle est soudée une couche à structure poreuse (8), de sorte que des canaux (10) sont formés entre les nervures (9) pour le passage du milieu cryogénique vaporisé,
**caractérisé en ce que**
pour atteindre et maintenir constante la température minimale, en particulier -40° C et -196° C, avec une faible perte de milieu cryogénique au niveau de la surface extérieure active de l'instrument cryogénique en contact avec un tissu biologique, en particulier un tissu cancéreux, l'élément à structure poreuse (8), par exemple en cuprum, dont le taux de porosité et la taille des pores, comprise dans l'intervalle de dimension d'environ 10 µm à 15 µm, croissent de l'intérieur vers l'extérieur, c'est-à-dire du côté amont vers le côté aval, est disposé dans le corps (1) de telle manière que les forces de capillarité de la structure poreuse (8) soient plus grandes que la pression de vapeur du milieu cryogénique éventuellement vaporisé au niveau des nervures (9) et que seul le milieu cryogénique vaporisé soit évacué dans la conduite cryogénique prévue pour le courant de retour (3) en passant par les canaux (10) existant entre les nervures (9), de sorte que la phase liquide contenue dans la couche poreuse (8) n'est pas influencée.

2. Instrument cryogénique selon la revendication 1,
**caractérisé en ce que**
la structure (8) est formée de segments de fils de cuivre à orientation aléatoire qui sont soudés les uns aux autres par le procédé de soudage par diffusion.

3. Instrument cryogénique selon la revendication 1,
**caractérisé en ce que**
il est réalisé sous la forme d'une aiguille cryogénique creuse, de préférence d'une longueur allant jusqu'à 150 mm et d'une largeur de 3 à 15 mm, le côté intérieur (12) du corps (1) sensiblement cylindrique est muni de nervures (9), de sorte que les canaux (10) présents entre les nervures (9) prévus pour la sortie du milieu cryogénique vaporisé sont ainsi formés, une couche ronde à structure poreuse (8) est soudée aux nervures (9) et les parois de la conduite (2) prévue pour le courant d'aller, sont munies, sur la longueur qui correspond à la longueur de congélation de l'aiguille cryogénique, d'une pluralité d'ouvertures de sortie de courant radiales (13) mutuellement opposées à travers lesquelles le milieu cryogénique pénètre dans la structure poreuse (8) en passant par un espace intermédiaire (14) qui est disposé entre la paroi de la conduite (2) prévue pour le courant d'aller et la structure poreuse (8).

4. Instrument cryogénique selon la revendication 3,
**caractérisé en ce que**
la conduite (2) est disposée avec jeu dans le volume intérieur de la structure poreuse (8).

5. Instrument cryogénique selon la revendication 3 ou 4,
**caractérisé en ce que**
le corps (1) présente sur son côté intérieur (12) des nervures (9) qui s'étendent sensiblement dans la direction axiale et entre lesquelles sont formés des canaux (10) destinés au retour du milieu cryogénique vaporisé.

6. Instrument cryogénique selon une des revendications 3 à 5,
**caractérisé en ce que**
pour éviter le capuchon de vapeur sur les nervures (9) au moment de l'établissement de la transition du milieu cryogénique de l'état liquide à l'état de vapeur, afin de pouvoir de cette façon atteindre aussi une température minimale sur la surface active de l'aiguille cryogénique sensiblement cylindrique, la surface active du corps sensiblement cylindrique (1) de l'aiguille cryogénique est munie extérieurement d'une isolation thermique (15) formée d'une couche mince, par exemple de 0,2-0,7 mm dans un instrument cryogénique de 4 mm de diamètre, l'épaisseur de la couche étant maintenue plus faible ou plus grande respectivement si le diamètre de l'aiguille cryogénique croît ou décroît respectivement et cette couche étant par ailleurs faite d'une matière isolante thermique à faible conduction thermique, par exemple, d'acier inoxydable, afin de remplir en supplément la condition nécessaire pour l'état de transition du milieu cryogénique, de sorte que le flux thermique atteignant le milieu cryogénique vaporisé dans la structure poreuse (8) est réduit et qu'on atteint la température minimale grâce à cette disposition complémentaire.

7. Instrument cryogénique selon la revendication 1,
**caractérisé en ce que**
il est réalisé sous la forme d'une sonde cryogénique et les éléments (8) possèdent une structure poreuse, les parois de la conduite (2) sont munies, dans le voisinage de l'embout (4), d'ouvertures de passage (13) mutuellement opposées à travers lesquelles le milieu cryogénique pénètre dans un espace intermédiaire (14) et, au niveau du côté intérieur (12) de l'embout (4) sont disposées des pointes (16) de forme conique de différentes longueurs, par exemple de 1 à 30 cm et en nombre variable, par exemple de 1 à 15, et munies intérieurement d'une structure poreuse (8) et extérieurement d'une paroi imperméable (17), les pointes (16) étant fixées de façon détachable au moyen d'un filetage (18).

8. Instrument cryogénique selon la revendication 1,
**caractérisé en ce que**
il est réalisé sous la forme d'un mammotome cryogénique dont l'embout (4) forme une seringue cryogénique qui, pour la biopsie et l'aspiration du tissu pathologique, en particulier d'un tissu de tumeur du sein, est munie à l'intérieur du corps (1), d'une isolation par le vide (19), d'un canal (20) isolé thermiquement, de préférence de 6 mm à 7 mm de diamètre, à paroi mince (21), de préférence de 0,2 à 0,3 mm, en acier inoxydable, en outre, le corps (1) est coudé selon un angle, de préférence de 15 ° à 160 °, et la conduite (2) prévue pour le courant d'aller du milieu cryogénique débouche dans l'élément à structure poreuse (8), de sorte que le côté intérieur (12) de la seringue cryogénique (4) congèle un résidu du tissu pathologique qui reste aussi bien dans le canal (20) que dans l'organe, par exemple dans le sein, après l'aspiration, **en ce que** la conduite (3) prévue pour le courant de retour du milieu cryogénique n'est pas reliée à l'élément à structure poreuse (8), et entoure le canal (20) en demi-cercle, de sorte que le canal (20) est maintenu libre en permanence pour différentes fonctions, en particulier pour la biopsie et l'aspiration du tissu pathologique.

9. Instrument cryogénique selon la revendication 1,
**caractérisé en ce que**
il est réalisé sous la forme d'une pince cryogénique possédant un corps (1) qui est muni d'une branche fixe et d'une branche mobile (22, 23) qui sont reliées entre elles par une articulation (24) et dont les surfaces actives (5) sont équipées de pointes (25) sur le côté extérieur (6), ainsi qu'une bouche possédant une partie mobile et une partie fixe (27, 28), la conduite (2) prévue pour le courant d'aller du milieu cryogénique et la conduite (3) prévue pour le courant de retour du milieu cryogénique étant disposées dans la partie fixe (28) de la bouche, un réchauffeur (29) isolé thermiquement se trouve au niveau du côté intérieur de la partie mobile (27) et de la partie fixe (28) de la bouche, et la partie fixe (28) de la bouche, qui n'est pas munie de pointes (25) est reliée à un échangeur de chaleur (30), de sorte qu'un pont (31) fait d'un conducteur de froid élastique, qui est formé d'un fil, de préférence de 4 à 8 mm de diamètre lui-même composé de plusieurs ressorts, de préférence de 20 à 60 µm de diamètre, en cuprum, est disposé entre l'échangeur de chaleur (30) et la partie mobile (27) de la bouche.
